# EUROPÄISCHE PATENTSCHRIFT

(11) **EP 1 506 781 B1**
(45) Veröffentlichungstag und Bekanntmachung des Hinweises auf die Patenterteilung: **23.02.2005**
(21) Anmeldenummer: 03025152.4
(22) Anmeldetag: 03.11.2003
(51) Int. Cl.: A61K 35/74, A61K 35/72, A61K 7/36

(54) **Vaginalpflegezusammensetzung**
Vaginal care composition
Composition pour soin vaginal

(43) Veröffentlichungstag der Anmeldung: 16.02.2005
(73) Patentinhaber: Volkmann, Peter-Hansen, 23569 Lübeck (DE)
(72) Erfinder: Volkmann, Peter-Hansen, 23569 Lübeck (DE)
(74) Vertreter: Meyer, Ludgerus A.

(56) Entgegenhaltungen:
- WO-A-03/071883
- US-A- 6 093 394
- US-A1- 2002 045 242
- DATABASE EPODOC [Online] EUROPEAN PATENT OFFICE, THE HAGUE, NL; "Healing preparations for early postnatal problems and mastitis of domestic animals" XP002272549 & CZ 288 454 A (MAHEL MIROSLAV MVDR) 13. Juni 2001 (2001-06-13)
- DATABASE WPI Section Ch, Week 199512 Derwent Publications Ltd., London, GB; Class B04, AN 1995-085329 XP002272550 & JP 07 010740 A (MEIJI MILK PROD CO LTD), 13. Januar 1995 (1995-01-13)
- PATENT ABSTRACTS OF JAPAN vol. 007, no. 077 (C-159), 30. März 1983 (1983-03-30) & JP 58 008018 A (AKIRA YAMAUCHI), 18. Januar 1983 (1983-01-18)
- MARTEAU P R: "Probiotics in clinical conditions" CLINICAL REVIEWS IN ALLERGY AND IMMUNOLOGY 2002 UNITED STATES, Bd. 22, Nr. 3, 2002, Seiten 255-273, XP009027205 ISSN: 1080-0549

## Beschreibung

Die vorliegende Erfindung betrifft eine Vaginalpflegezusammensetzung. Insbesondere betrifft die Erfindung eine Vaginalpflegezusammensetzung, die in Form eines Ovulums, einer Creme oder Salbe oder mit einem Tampon direkt in die Vagina eingebracht wird oder auf einer Slipeinlage vor die Vagina gebracht wird.

Es ist seit langem bekannt, dass Schleimhäute sehr empfindlich sein können. Die Schleimhaut der Scheide ist dabei besonders empfindlich für Irritationen oder auch Entzündungen. Entsprechend veranlagte Frauen reagieren zum Beispiel nach dem Besuch eines Schwimmbades oder eines anderen Gewässers oder nach dem Geschlechtsverkehr mit Irritationen, schmerzhaften Veränderungen oder sogar Entzündungen im Bereich der Vaginalschleimhaut. Auch während hormoneller Umstellungen, z.B. unter einer Pilleneinnahe ("Antibabypille"), in der Schwangerschaft, der Stillzeit oder während der Menopause kann die Vaginalschleimhaut gereizt reagieren. Ähnlich kommt es z.B. auch bei einer systemischen Antibiotika-Behandlung zu Irritationen der Schleimhäute, insbesondere der Vaginalschleimhaut. Ein weiterer irritierender Faktor für die Scheidenflora ist der zunehmend zu beobachtende Ausfluß aus der Scheide, der ursächlich aus dem Bauchraum kommt und durch eine unphysiologische Zusammensetzung der Sekrete das normale Gleichgewicht der Vaginalbesiedelung stören kann.

Die daraus resultierenden Irritationen der Vaginalschleimhaut sind nicht nur schmerzhaft, sondern können sich auch zu aufsteigenden Entzündungen entwickeln, die weitere Organe oder den gesamten Körper der Frau betreffen können. Tatsächlich sind mehrere Milliarden Frauen jährlich von derartigen Irritationen befallen, die das Lebensgefühl deutlich beeinträchtigen können.

Eine Theorie, die erklären könnte, warum sich Irritationen und auch entzündliche Reaktionen entwickeln können, involviert die Bildung von Biofilmen. Solche Biofilme können an der Entstehung von / Besiedelung mit pathogenen Keimen beteiligt sein und/oder diese unterstützend beeinflussen oder auch hemmen. Die Verhinderung eines Entstehens solcher pathologischer Biofilme wäre also wünschenswert. Der Aufbau eines physiologischen Schleimhaut-Schutzfilms ist das Ziel dieser Anmeldung.

Daher sollte die Schleimhaut derartig gepflegt werden, dass sie Umwelteinflüssen besser widerstehen kann und keine Umgebung bietet, in der sich pathologische Biofilme entwickeln können. Eine solche Pflege wird z.B. für die äußere Haut des Körpers weit verbreitet schon seit langem praktiziert, z.B. durch die Verwendung pflegender Öle oder Cremes, vorzugsweise regelmäßig, insbesondere aber nach besonderen Belastungen, denen die Haut ausgesetzt wird, z.B. nach einem Sonnenbad oder nach dem Duschen oder Schwimmen.

Zum Schutz vor Infektionen bei dem Besuch von Schwimmbädern gibt es bereits Tampons, die solchen Infektionen vorbeugen sollen. Diese "Symbiofem" Tampons, die z.B. auf der Website www.symbiofem.com zu finden sind, sind mit Vaselineöl getränkt. Allerdings bieten diese Tampons lediglich eine rein mechanische Sperrschicht, die keineswegs zur Pflege der Vaginalschleimhaut beträgt. Im Gegenteil ist es fraglich, ob der Kontakt mit hochkonzentriertem Vaselineöl der Schleimhautumgebung zuträglich ist. Ferner behindert das Vaselineöl die notwendige Ausdehnung eines Tampons, so dass eine exakte Anpassung an die Körperform der Trägerin kaum erfolgen kann und im Endeffekt dennoch Wasser durchgelassen werden wird.

Es ist daher wünschenswert, dass es auch für die Vaginalschleimhaut Pflegeprodukte gäbe, mit denen diese ähnlich gepflegt und geschützt werden könnte.

Es ist daher die Aufgabe der vorliegenden Erfindung, eine Vaginalpflegezusammensetzung bereitzustellen, mit der die Schleimhaut der Vagina geschützt werden kann.

Diese Aufgabe wird durch die in den unabhängigen Ansprüche angegebene Erfindung gelöst. Vorteilhafte Weiterbildungen sind in den abhängigen Ansprüchen angegeben.

Gemäß der Erfindung wird daher eine Vaginalpflegezusanunensetzung angegeben, umfassend lebensfähige Lactobacillus und/oder Bifidobakterium-Keime, vorzugsweise Lactobacillus bifidus; nicht lebensfähige Saccharomyces-Kulturen, vorzugsweise Saccharomyces cerevisiae; Saccharid(e); Vitamin-A-Retinol, und Zink.

Lactobacillus ist der bevorzugte verwendete Mikrooorganismus. Er kann vorzugsweise gewählt werden aus der Gruppe, bestehend aus: Lactobacillus bifidus, Lactobacillus rhamnosus, Lactobacillus acidophilus, Lactobacillus crispatus, Lactobacillus fermentum, Lactobacillus plantarum, Lactobacillus casei, Lactobacillus paracasei, Lactobacillus jensenii, Lactobacillus gasseri, Lactobacillus cellobiosis, Lactobacillus brevis, Lactobacilllus delbrueckii, Lactobacillus rogosae und Lactobacillus bifidum.

Ferner kann auch Bifidobakterium allein oder in Mischung mit dem obigen Lactobacillus verwendet werden. Er wird vorzugsweise gewählt aus: Bifidobakterium bifidum, Bifidobakterium breve, Bifidobakterium adolescentis oder Bifidobakterium longum.

Die obigen Arten können allein oder als Mischung von zwei oder mehr Arten verwendet werden. Vorzugsweise sind die Mikroorganismen in der Zusammensetzung in lyophilisierter oder wärme-getrockneter Form enthalten. Jedenfalls müssen sie in der Zusammensetzung in einer lebensfähigen Weise enthalten sein, die es ihnen ermöglicht, bei Kontakt mit Feuchtigkeit (die z.B. der vaginalen Umgebung entstammt), zu wachsen und sich zu vermehren.

Die bevorzugte Saccharomyces Art ist Saccharomyces cerevisiae. Diese ist als Kultur, z.B. in getrockneter Form, in der Zusammensetzung enthalten. Die Saccharomyces Kultur darf nicht lebensfähig sein, um nicht in Konkurrenz zu Lactobacillus bzw. Bifidobakterium zu treten; sie dient als Startmaterial für das Wachstum der Lactobacillen/Bifidobakterien und als Nährboden für deren weitere Vermehrung.

Die Vaginalpflegezusammensetzung ist insbesondere bevorzugt dadurch gekennzeichnet, dass zusätzlich Selen, insbesondere selenhaltige Hefe, enthalten ist, vorzugsweise in einer Menge von 0,001 - 0,06 Gewichtsprozent Selen. Das Selen ist ein antioxidatives Spurenelement mit Zellschutzeigenschaften, dass das Wachstum von Lactobacillen und Bifidobakterien besonders fördert und daher deren Entwicklung gegenüber derer anderer Keime in der Vagina begünstigt.

Die Vaginalpflegezusammensetzung ist ferner vorzugsweise dadurch gekennzeichnet, dass zusätzlich Melaleuca aetheroleum, vorzugsweise in einer Menge von 0,5 bis 10 Gewichtsprozent, und/oder Kamillenblütenauszug, vorzugsweise in einer Menge von 0,01 bis 5 Gewichtsprozent, enthalten ist. Beide Substanzen unterstützen die Pflege der vaginalen Schleimhaut durch ihre entzündungslindemden und pflegenden Eigenschaften.

Die Vaginatpflegezusammensetzung ist außerdem in einer besonders bevorzugten Ausführungsform dadurch gekennzeichnet, dass zusätzlich D-alpha-Tocopherol, vorzugsweise in einer Menge von 0,5 bis 5 Gewichtsprozent, und/oder Pantothenat, vorzugsweise in einer Menge von 0,1 bis 3 Gewichtsprozent, enthalten ist. Das Tocopherol wirkt gemäß einer bevorzugten Ausführungsform als Antioxidans für durch die Zusammensetzung eingebrachte Öle sowie als Zellschutz für Haut und Schleimhaut. Pantothenat ist ein. Vitamin, dass wiederum das Wachstum von Lactobacillen und/oder Bifidobakterium gegenüber anderen Keimen fördert und (so) durch seine Vitamineigenschaften besonders zur Pflege der Vaginalschleimhaut beiträgt.

Gemäß einer weiteren bevorzugten Ausführungsform enthält die Vaginalpflegezusammensetzung zusätzlich Duftstoffe, vorzugsweise natürliche Duftstoffe, insbesondere bevorzugt Lavendelöl in einer Menge von 0,5 bis 10 Gewichtsprozent, enthalten sind. Die Duftstoffe können z.B. enthalten sein um die Pflegezusammensetzung von einer potentiellen Verwenderin besser angenommen zu werden.

Als Basis kann die Vaginalpflegezusammensetzung beispielsweise Kakaobutter oder einen Hartfett-Träger umfassen. Derartige Träger sind dem Fachmann bekannt. Sie zeichnen sich insbesondere durch ihre gute Verträglichkeit im Bereich der Vagina aus und führen dort nicht zu Irritationen oder etwaigen allergischen Reaktionen.

Im folgenden wird eine beispielhafte Vaginalpflegezusammensetzung angegeben, die Bestandteile in den folgenden Mengen enthält (im folgenden sind Gewichtsprozent immer bezogen auf die Gesamtzusammensetzung):
- Lactobacillus/Bifidobakterium : 10⁷ - 10¹⁰ Keime/g, 3 - 5% Zusammensetzung
- Saccharomyces: 0,1 - 40 Gewichtsprozent
- Saccharid(e): 1 - 40 Gewichtsprozent
- Vitamin A: 0,01 - 0,5 Gewichtsprozent
- Zink: 0,2 - 10 Gewichtsprozent.

Vorzugsweise wird das Saccharid für die Vaginalpflegezusammensetzung gewählt aus: Oligofructose, Inulin und/oder Lactose, wobei Oligofructose bevorzugt wird. Es ist auch möglich eine Mischung aus zwei oder mehreren der oben angegebenen Saccharide zu verwenden. Die Saccharide dienen insbesondere als Startmaterialien für die Entwicklung der Lactobacillen und Bifidobakterien. Diese verwerten die Saccharide und haben dadurch einen Wachstumsvorteil gegenüber anderen Keimen.

Die Vaginalpflegezusammensetzung kann so zusammengesetzt sein, dass das Zink in Form von Zinksulfat und/oder Zinkgluconat eingesetzt wird. Ferner kann die Vaginalpflegezusammensetzung eine Formulieren aufweisen, in der das Vitamin A als Retinylacetat und/oder Retinylgluconat eingesetzt wird. Diese Formen der Zumischung von Zink bzw. Vitamin A sind dem Fachmann bekannt. Andere Formen von Vitamin A bzw. Zink können gewählt werden, sofern dadurch die Funktion der Zusammensetzung als Pflegemittel für die Vaginalschleimhaut nicht beeinträchtigt wird.

Die Zusammensetzung kann je nach Art auch weitere konventionelle Bestandteile enthalten, solange diese die Funktion einer Pflegezusammensetzung nicht beeinträchtigen. Diese können z.B. gewählt sein aus Bindemitteln, Exzipientien, Lösungsmitteln, Stabilisatoren, Gleitmitteln, Desintegrationsmitteln, und Trägern, wie z.B. feste oder flüssige Träger.

Gemäß einem Hauptaspekt der vorliegenden Erfindung wird die Vaginalpflegezusammensetzung wie oben definiert für die Pflege der Vaginalschleimhaut und/oder zur Vorbeugung von Infektionen derselben durch Bakterien, Pilze und/oder Viren verwendet. Die besondere Zusammensetzung ermöglicht es, die Vaginalschleimhaut zu pflegen ohne sie in negativer Weise zu beeinflussen oder zu beeinträchtigen. Diese Pflege kann auf regelmäßiger Basis erfolgen oder nach Bedarf, z.B. in Phasen hormoneller Umstellungen, wie z.B. während einer Schwangerschaft, während der Stillzeit, in der Menopause oder postmenopausal. Es ist auch möglich, die Schleimhaut ganz gezielt zu pflegen, z.B. vor und/oder nach Besuchen in öffentlichen Gewässern, wie z.B. Badeseen oder Schwimmbädern.

Dabei wird die Vaginalpflegezusammensetzung vorzugsweise direkt auf die Vaginalschleimhaut aufgebracht. Dies kann z.B. dadurch bewirkt werden, dass die Zusammensetzung als Ovulum oder Creme bzw. Salbe in die Vagina eingeführt und dadurch in direkten Kontakt mit der Schleimhaut gebracht wird. Ferner kann die Zusammensetzung in einem Tampon enthalten sein, der dann in die Vagina eingeführt wird und dadurch die Zusammensetzung mit der Schleimhaut kontaktiert. Schließlich ist die Zusammensetzung gemäß einer weiteren bevorzugten Ausführungsform in einer Slipeinlage enthalten. Die Zusammensetzung wird jeweils durch Feuchtigkeit aktiviert, z.B. die Feuchtigkeit der üblichen vaginalen Umgebung. "Aktivieren" in diesem Zusammenhang bedeutet, dass die enthaltenen Lactobacillen bzw. Bifidobakterien zu wachsen und sich zu vermehren beginnen.

Daher betrifft die vorliegende Erfindung gemäß einer besonders bevorzugten Ausführungsform ein pflegendes Ovulum, umfassend die Vaginalpflegezusammensetzung wie oben definiert. Vorzugsweise umfasst das pflegende Ovulum - mit einem Gewicht von 2 - 5 g, vorzugsweise 3 g pro Ovulum, das folgende pro Gramm: # 10⁷ - 10⁹, vorzugsweise 10⁸ Keime/g 5 % Lactobacillus bifidus,
# 1 - 10, vorzugsweise 2,5 Gew % Saccharomyces cerevisiae,
# 0,05 - 0,3, vorzugsweise 0,1 Gew % Vitamin A,
# 1 - 3, vorzugsweise 1,8 Gew % D-alpha-Tocopherol,
# 0,4 - 1,5, vorzugsweise 0,9 Gew % Pantothenat,
# 0,5 - 5, vorzugsweise 0,8 Gew % Zink,
# 0,002 - 0,02, vorzugsweise 0,005 Gew % Selen,
# 1 - 5, vorzugsweise 3 Gew % Melaleuca aetheroleum,
# 1 *-* 5, vorzugsweise 3 Gew % Lavendelöl,
# 0,2 - 3, vorzugsweise 1 Gew % Kamillenblütenöl-Auszug und/oder
# 2 - 20, vorzugsweise 5 Gew % Oligofructose.

Im folgenden wird die bevorzugte Zusammensetzung eines Ovulums in tabellarischer Form mit Angabe der jeweiligen kombinatorischen Wirkung zusammengefasst:

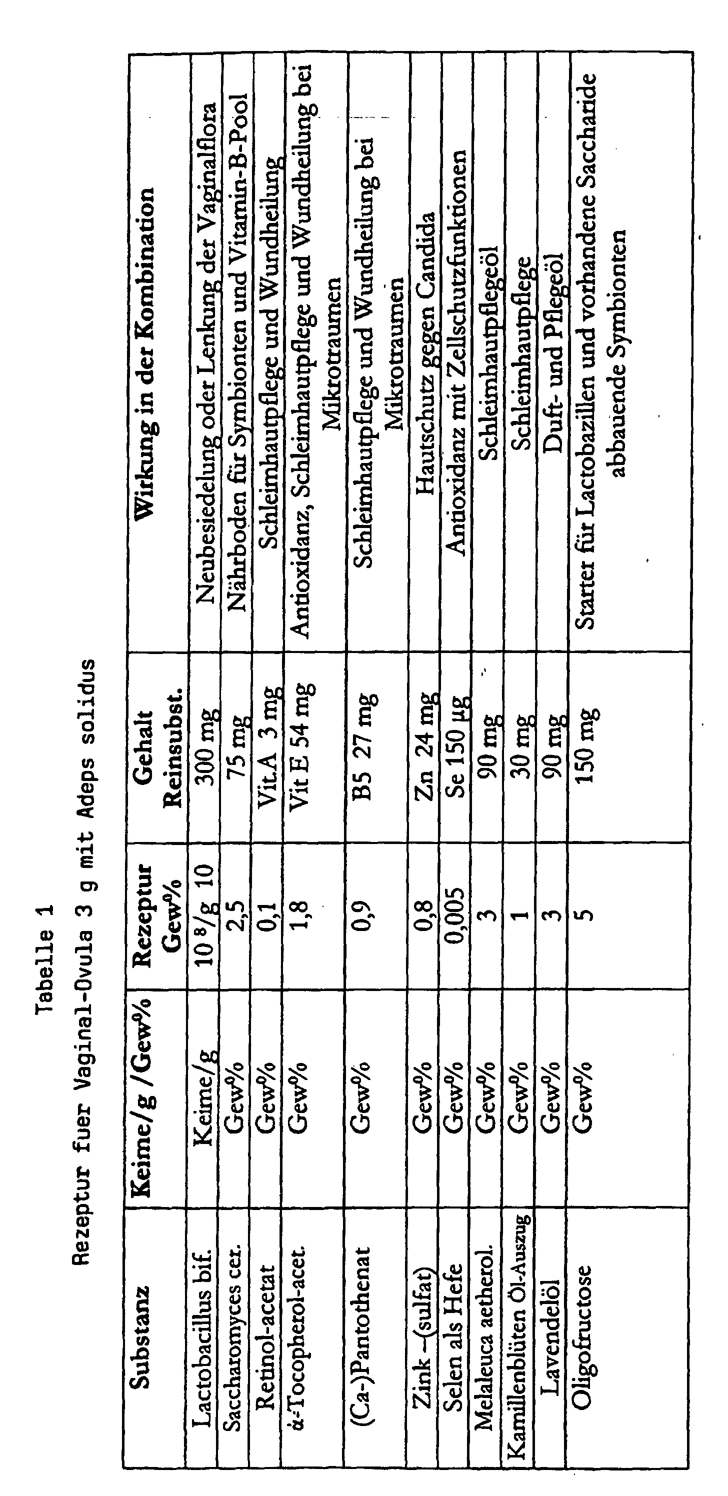

Dabei soll die Bezeichnung "Ovulum" im Sinne der vorliegenden Erfindung die dem Fachmann unter dieser Bezeichnung bekannten festen Formen, wie z.B Vaginalzäpfchen, zur Einbringung in die Vagina, als auch Schaumtabletten oder Depotimplantate umfassen. Bevorzugt umfasst ein Ovulum die konventionellen Basen, wie z.B. Whitepsol, Massa Estarinum, Kokosnussfett, Glycerin-Gelatine-Massen Glycerin-Seifen oder Polyethylenglycol.

Gemäß einer weiteren bevorzugten Ausführungsform betrifft die vorliegende Erfindung eine Pflegecreme, umfassend die Vaginalzusammensetzung wie oben definiert. Vorzugsweise umfasst die Pflegecreme
# 10⁷ bis 10⁹, vorzugsweise 10⁸ Keime/g Lactobacillus bifidus, 5%,
# 1 - 10, vorzugsweise 2,5 Gew. % Saccharomyces cerevisiae,
# 0,05 bis 0,3 Gew.%, vorzugsweise 0,1 Gew % Vitamin A,
# 1 - 3 Gew%, vorzugsweise 1,5 Gew% D-alpha-Tocopherol,
# 0,4 bis 1,5 Gew%, vorzugsweise 0,7 Gew% Pantothenat,
# 1 - 6 Gew%, vorzugsweise 3 Gew% Zink,
# 1 - 5 Gew%, vorzugsweise 2 Gew% Melaleuca aetheroleum,
# 0,05 - 3 Gew%, vorzugsweise 1 Gew% Kamillenblütenauszug,
# 2-20, vorzugsweise 5 Gew % Oligofructose
# und/oder 1 -5 Gew%, vorzugsweise 3 Gewichtsprozent Lavendelöl.

Gemäß einer weiteren bevorzugten Form umfasst die Pflegecreme weitere konventionelle Creme- oder Salben-Bestandteile, insbesondere Bestandteile, gewählt aus der folgenden Gruppe: Sonnenblumenöl, Tegomuls 90 S, Cetylalkohol, Cetaceum artificiale, Polysorbat 80, Methyl-4-hydroxybenzoat, Propyl-4-hydroxybenzoat, Sesamöl, Wollwachs, Gelbes Wachs, Wollwachsalkohole, Glycerin, Glycerinester, Palmitinsäureester, weißes Vaselin, dünnflüssiges Wachs, Olivenöl und/oder destilliertes Wasser.

Die Bezeichnung "Creme" im Sinne der vorliegenden Erfindung umfasst Cremes, Salben als auch Emulsionen und aus Gelatine freizusetzende flüssige Zusammensetzungen.

Eine besonders bevorzugte Salbengrundlage ist die folgende:
Sonnenblumenöl, Tegomuls 90 S, Cetylalkohol, Cetaceum artificiale, Polysorbat 80, Methyl-4-hydroxybenzoat und Propyl-5-hydroxybenzoat in destilliertem Wasser oder
Sesamöl, Wollwachs, gelbes Wachs, Wollwachsalkohole oder
Glycerin, Glycerinester, Palmitinsäureester, weißes Vaselin, dünnflüssiges Wachs oder
Gelbes Wachs, Olivenöl.

Die Erfindung betrifft gemäß einer weiteren besonders bevorzugten Ausführungsform einen pflegenden Vaginaltampon, umfassend die Vaginalpflegezusammensetzung wie oben definiert. Besonders bevorzugt kann der pflegende Vaginaltampon folgendes umfassen (umfassend 2 - 5, vorzugsweise 3 g der Vaginalpflegezusammensetzung pro Tampon), (pro Gramm der Zusammensetzung):
# 10⁷ - 10⁹, vorzugsweise 10⁸ Keime/g Lactobacillus bifidus, 5%,
# 1 - 10, vorzugsweise 2,5 Gew % Saccharomaces cerevisiae,
# 0,05 - 0,3, vorzugsweise 0,1 Gew % Retinol-Acetat,
# 1 bis 3, vorzugsweise 1,5 Gew % D-alpha-Tocopherol,
# 1 - 6, vorzugsweise 0,8 Gew % Zink,
# 1 - 5, vorzugsweise 2 Gew % Melaleuca aetheroleum, und/oder
# 2 - 20, vorzugsweise 3 Gew % Oligofructose.

Damit die Zusammensetzung die Vaginalschleimhaut möglichst einfach und schnell erreichen kann, sollte der pflegende Vaginaltampon die Vaginalpflegezusammensetzung auf einer oberflächlichen Schicht des Tampons enthalten, wobei die Zusammensetzung beispielsweise durch Sprüh- oder Tauchverfahren aufgebracht wird. Optional kann die Zusammensetzung auch den gesamten Tampon durchdringen. Die Vaginalpflegezusammensetzung kann gemäß einer besonders bevorzugten Ausführungsform auch ausschließlich in einer Schicht direkt unter der äußeren Deckschicht des Tampons enthalten sein.

Die Vaginalpflegezusammensetzung der vorliegenden Erfindung kann auf jeden dem Fachmann bekannten Tampon aufgebracht werden oder dieser kann damit getränkt werden. Beispielhaft besteht der Tampon aus einer in sich gedrehten Form, mit außen, d.h. zum Körper der Trägerin hin, gerichteten Rillen oder Vertiefungen, die das Ableiten von Flüssigkeit in das Innere des Tampons erleichtern. Der Tampon weist ferner vorzugsweise eine äußere, flüssigkeitsableitende Schicht auf und eine innere Flüssigkeitsspeicherschicht. Ferner enthält er vorteilhaft durch Flüssigkeit quellende Materialien, damit sich der Tampon an die Körperform der jeweiligen Trägerin anpassen kann. Beispielsweise kann der Tampon eine Deckschicht, umfassend Zellulose aufweisen, die bei Kontakt mit Feuchtigkeit quillt und kurzfristig ein Gel bildet, das das Einführen erleichtert. Ein derartiger Tampon ist dem Fachmann bekannt.

Gemäß einer besonders bevorzugten Ausführungsform ist die Vaginalpflegezusammensetzung gemäß der vorliegenden Erfindung in außen gelegenen Schicht des Tampons enthalten, ohne dass die Erfindung hierauf begrenzt wäre.

Gemäß einer weiteren bevorzugten Ausführungsform betrifft die vorliegende Erfindung eine pflegende Slipeinlage, umfassend die Vaginalpflegezusammensetzung wie oben definiert. Bevorzugt umfasst die pflegende Slipeinlage, umfassend 2 - 4g, vorzugsweise 3g der Vaginalpflegezusammensetzung pro Slipeinlage, in 1 g der Zusammensetzung:
# 10⁷ - 10⁹, vorzugsweise 10⁸ Keime/g Lactobacillus bifidus, 5%,
# 1 - 10, vorzugsweise 2,5 Gew % Saccharomaces cerevisiae,
# 0,05 - 0,3, vorzugsweise 0,1 Gew % Retinol-Acetat,
# 1 bis 3, vorzugsweise 1,5 Gew % D-alpha-Tocopherol,
# 1 - 6, vorzugsweise 3 Gew % Zink, und/oder
# 2 - 20, vorzugsweise 3 Gew % Oligofructose.

Die pflegende Slipeinlage kann die Vaginalpflegezusammensetzung auf einer oberen Schicht enthalten, vorzugsweise aufgebracht durch ein Sprühverfahren. Es ist auch möglich die pflegende Slipeinlage so auszubilden, dass die Vaginalpflegezusammensetzung ausschließlich in einer Schicht direkt unter der Deckschicht der Slipeinlage enthalten ist.

Die Vaginalpflegezusammensetzung kann auf jede dem Fachmann bekannte Slipeinlage aufgebracht/eingebracht werden. Beispielhaft kann die Slipeinlage aus einer feuchtigkeitsdurchlässigen Deckschicht, einer die Feuchtigkeit ableitenden, darunter angeordneten Schicht, einer Feuchtigkeitsspeicherschicht, die unter der die Feuchtigkeit ableitenden Schicht angeordnet ist und einer flüssigkeitsundurchlässigen Rückschicht bestehen. Die Deckschicht kann z.B. Poren aufweisen, die die Ableitung von Flüssigkeit vereinfachen und die Belüftung verbessern. Ferner können maskierende Substanzen enthalten sein, die das Aussehen der Slipeinlage verbessern. Besonders bevorzugt ist die Vaginalpflegezusammensetzung der vorliegenden Erfindung in einer Schicht unterhalb der Deckschicht enthalten, ohne dass die Erfindung hierauf begrenzt wäre.

In einer weiteren Ausführungsform kann die vorliegende erfinderische Pflegezusammensetzung auch als Harnröhrenstick zur Pflege der Harnröhrenschleimhaut bei Partnerpflege für Herren verwendet werden. Die Pflegezusammensetzung kann in dieser Ausführungsform beispielsweise in ein Harnröhrenstäbchen aus Adeps solidus eingebettet werden. Dieses Stäbchen sollte eine Länge von 10 bis 30 cm, vorzugsweise 25 cm, und einen Durchmesser von 2,5 bis 7 mm, vorzugsweise 4,5 mm aufweisen. Es kann durch Sollbruchstellen segmentiert sein, die z.B. in Abständen von ca. 5 cm angeordnet sind.

Eine bevorzugte Zusammensetzung für das Hamröhrenstäbchen besteht aus Lactobacillus bifidus-Keimen, mit Saccharomyces cerevisiae und Oligofructose als Starter. Ferner sollten Vitamin A, Alpha-Tocopherol, Pantothenat, Zink, Selen, Melaleuca aetheroleum und Duftstoffe enthalten sein. Vorzugsweise enthält das Harnröhrenstäbchen pro gramm:
# 10⁷ - 10⁹, vorzugsweise 10⁸ Keime Lactobacillus bifidus,
# 1 - 10, vorzugsweise 2,5 Gew % Saccharomaces cerevisiae,
# 0,05 -0,3, vorzugsweise 0,1 Gew % Retinol-Acetat,
# 1 bis 3, vorzugsweise 1,5 Gew % D-alpha-Tocopherol,
# 1 - 6, vorzugsweise 3 Gew % Zink, und/oder
# 2 - 20, vorzugsweise 3 Gew % Oligofructose.

Die Dosierung der erfindungsgemäßig Vaginalpflegezusammensetzung hängt von dem Fachmann bekannten Faktoren ab; üblicherweise sollte die Schleimhaut 1 bis 3 mal täglich mit bis zu 3 g der Zusammensetzung gepflegt werden; alternativ direkt vor und nach einer besonderen Belastung, wie Schwimmen oder Geschlechtsverkehr. Eine langfristige Pflege kann mit z.B. 6 Ovula pro Zyklus durchgeführt werden.

### BEISPIELE

### 1. Salbe

### A. Herstellung, Zusammensetzung

Es wurde eine Salbe hergestellt, die die folgende Zusammensetzung hatte:
- 10⁸ Keime /g Lactobacillus bifidus (/g) 10% der erfinderischen Zusammensetzung
- 2.5 Gew % Saccharomyces cerevisiae, als Nährboden
- 0,1 Gew % Retinylacetat
- 1,8 Gew % D-alpha-Tocopherol
- 0, 9 Gew % Pantothenat
- 0,8 Gew % Zink
- 0,005 Gew % Selen (als selenhaltige Hefe)
- 3 Gew % Melaleuca aetheroleum
- 1 Gew % Kamillenblütenauszug
- 3 Gew % Lavendelöl
- 5 Gew % Oligofructose
gemischt nach den dem Fachmann bekannten Verfahren zur Herstellung von Salben in einer Mischung aus gelbem Wachs und Olivenöl.

### B. Anwendung

Eine Dame (25 Jahre, mit Pilleneinnahme), die in den 5 vorhergegangen Jahren jeweils im Sommer mehrere Vaginalmykosen hatte, verwendete die obige Salbe nach der folgenden Vorschrift:
Einmal täglich abends wurden je 3 g der Salbe auf der Vaginalschleimhaut verteilt. Die Pflege wurde im Mai begonnen. Während der folgenden Sommermonate traten keine Mykosen auf.

### 2. Ovulum

### A. Herstellung, Zusammensetzung

Jedes Ovulum wog 3 g und wurde gemäß den dem Fachmann bekannten Verfahren für die Herstellung von Ovula aus der folgenden Mischung hergestellt:
- 10⁸ Keime Lactobacillus bifidus/g, 5 % der erfinderischen Zusammensetzung
- 2.5 Gew % Saccharomyces cerevisiae, als Kultur
- 0,1 Gew % Retinylacetat
- 1,5 Gew % D-alpha-Tocopherol
- 0,7 Gew % Pantothenat
- 0,8 Gew % Zink
- 0,005 Gew % Selen (als selenhaltige Hefe)
- 2 Gew % Melaleuca aetheroleum
- 1 Gew % Kamillenblütenauszug
- 3 Gew % Lavendelöl
- 3 Gew % Oligofructose.

Die Zutaten wurden in Adeps solidus mit einem Schmelzpunkt von 34,5 Grad Celsius eingemischt.

### B. Anwendung

Eine Dame (43 Jahre), die in den vorhergehenden Jahren ca. 3 - 5 Vaginalmykosen pro Jahr hatte, nahm, beginnend im Januar, die Ovula nach der folgenden Vorschrift:
Einmal täglich abends wird ein Ovulum eingeführt.

Daraufhin war sie für den Rest des Jahres frei von Vaginal-Infektionen.

Die obigen Beispiele und die Beschreibung sollen die Erfindung weiter erläutern, sind jedoch nicht dazu bestimmt, ihren Umfang zu beschränken, der durch die Ansprüche definiert ist.

## Patentansprüche

1. Vaginalpflegezusammensetzung, umfassend lebensfähige Lactobacillus und/oder Bifidobakterium-Keime, vorzugsweise Lactobacillus bifidus; nicht lebensfähige Saccharomyces-Kulturen, vorzugsweise Saccharomyces cerevisiae; Saccharid(e); Vitamin-A-Retinol, und Zink.

2. Vaginalpflegezusammensetzung gemäß Anspruch 1, **dadurch gekennzeichnet, dass** zusätzlich Seien, insbesondere selenhaltige Hefe, enthalten ist, vorzugsweise in einer Menge von 0,001 - 0,06 Gewichtsprozent Selen.

3. Vaginalpflegezusammensetzung gemäß Anspruch 1 und/oder 2, **dadurch gekennzeichnet, dass** zusätzlich Melaleuca aetheroleum, vorzugsweise in einer Menge von 0,5 bis 10 Gewichtsprozent, und/oder Kamillenblütenauszug, vorzugsweise in einer Menge von 0,01 bis 5 Gewichtsprozent, enthalten ist.

4. Vaginalpflegezusammensetzung gemäß einem oder mehreren der Ansprüche 1 bis 3, **dadurch gekennzeichnet, dass** zusätzlich D-alpha-Tocopherol, vorzugsweise in einer Menge von 0,5 bis 5 Gewichtsprozent, und/oder Pantothenat, vorzugsweise in einer Menge von 0,1 bis 3 Gewichtsprozent, enthalten ist.

5. Vaginalpflegezusammensetzung gemäß einem oder mehreren der vorstehenden Ansprüche, **dadurch gekennzeichnet, dass** zusätzlich Duftstoffe, vorzugsweise natürliche Duftstoffe, insbesondere bevorzugt Lavendelöl in einer Menge von 0,5 bis 10 Gewichtsprozent, enthalten sind.

6. Vaginalpflegezusammensetzung gemäß einem oder mehreren der vorstehenden Ansprüche, **dadurch gekennzeichnet, dass** sie als Basis Kakaobutter oder einen Hartfett-Träger umfasst.

7. Vaginalpflegezusammensetzung gemäß Anspruch 1, **dadurch gekennzeichnet, dass** die Bestandteile in den folgenden Mengen enthalten sind:
- Lactobacillus/Bifidobakterium : 10⁷ - 10¹⁰ Keime/g Zusammensetzung, 5%
- Saccharomyces: 0,1 - 40 Gewichtsprozent
- Saccharid(e): 1 - 40 Gewichtsprozent
- Vitamin A: 0,01 - 0,5 Gewichtsprozent
- Zink: 0,2 - 10 Gewichtsprozent.

8. Vaginalpflegezusammensetzung gemäß einem oder mehreren der vorstehenden Ansprüche, wobei das Saccharid (die Saccharide) gewählt ist aus: Oligofructose, Inulin und/oder Lactose, wobei Oligofructose bevorzugt wird.

9. Vaginalpflegezusammensetzung gemäß einem oder mehreren der vorstehenden Ansprüche, wobei das Zink in Form von Zinksulfat und/oder Zinkgluconat eingesetzt wird.

10. Vaginalpflegezusammensetzung gemäß einem oder mehreren der vorstehenden Ansprüche, wobei das Vitamin-A als Retinylacetat und/oder Retinylgluconat eingesetzt wird.

11. Verwendung der Vaginalpflegezusammensetzung gemäß einem oder mehreren der vorstehenden Ansprüche für die Pflege der Vaginalschleimhaut und zur Vorbeugung von Infektionen derselben durch Bakterien, Pilze und/oder Viren.

12. Verwendung der Vaginalpflegezusammensetzung gemäß Anspruch 11, wobei die Zusammensetzung direkt auf die Vaginalschleimhaut aufgebracht wird.

13. Pflegendes Ovulum, umfassend die Vaginalpflegezusammensetzung gemäß einem der Ansprüche 1 bis 10.

14. Pflegendes Ovulum gemäß Anspruch 13, mit einem Gewicht von 3 g pro Ovulum, umfassend pro Gramm:
# 10⁷ - 10⁹, vorzugsweise 10⁸ Keime Lactobacillus bifidus/g, 5%
# 1 - 10, vorzugsweise 2,5 Gew % Saccharomyces cerevisiae,
# 0,05 - 0,3, vorzugsweise 0,1 Gew % Vitamin A,
# 1 - 3, vorzugsweise 1,8 Gew % D-alpha-Tocopherol,
# 0,4 - 1,5, vorzugsweise 0,9 Gew % Pantothenat,
# 0,5 - 5, vorzugsweise 0,8 Gew % Zink,
# 0,002 - 0,02, vorzugsweise 0,005 Gew % Selen,
# 1 - 5, vorzugsweise 3 Gew % Melaleuca aetheroleum,
# 1 - 5, vorzugsweise 3 Gew % Lavendelöl,
# 0,2 - 3, vorzugsweise 1 Gew % Kamillenblütenöl-Auszug und/oder
# 2 - 20, vorzugsweise 5 Gew % Oligofructose.

15. Pflegendes Ovulum gemäß Anspruch 13 und/oder 14, wobei das Ovulum auf Basis von Kokosfett, Glycerin-Gelatine oder Polyethylenglycol aufgebaut ist.

16. Pflegendes Ovulum gemäß einem oder mehreren der Ansprüche 13 bis 15, wobei das Ovulum weiterhin konventionelle Ovula-Bestandteile umfasst, insbesondere Kakaobutter oder Hartfette.

17. Pflegecreme, umfassend die Vaginalzusammensetzung gemäß einem der Ansprüche 1 - 10.

18. Pflegecreme gemäß Anspruch 17 umfassend
# 10⁷ bis 10⁹, vorzugsweise 10⁸ Keime Lactobacillus bifidus/g, 5%,
# 1 - 10, vorzugsweise 2,5 Gew. % Saccharomyces cerevisiae,
# 0,05 bis 0,3 Gew.%, vorzugsweise 0,1 Gew % Vitamin A,
# 1 - 3 Gew%, vorzugsweise 1,5 Gew% D-alpha-Tocopherol,
# 0,4 bis 1,5 Gew%, vorzugsweise 0,7 Gew% Pantothenat,
# 1 - 6 Gew%, vorzugsweise 3 Gew% Zink,
# 1 - 5 Gew%, vorzugsweise 2 Gew% Melaleuca aetheroleum,
# 0,05 - 3 Gew%, vorzugsweise 1 Gew% Kamillenblütenauszug,
# 2-20, vorzugsweise 5 Gew % Oligofructose
# und/oder 1 -5 Gew%, vorzugsweise 3 Gewichtsprozent Lavendelöl.

19. Pflegecreme gemäß Anspruch 17 und/oder 18, wobei die Creme weiterhin konventionelle Creme- oder Salben-Bestandteile umfasst, insbesondere Bestandteile, gewählt aus der folgenden Gruppe: Sonnenblumenöl, Tegomuls 90 S, Cetylalkohol, Cetaceum artificiale, Polysorbat 80, Methyl-4-hydroxybenzoat, Propyl-4-hydroxybenzoat, Sesamöl, Wollwachs, Gelbes Wachs, Wollwachsalkohole, Glycerin, Glycerinester, Palmitinsäureester, weißes Vaselin, dünnflüssiges Wachs, Olivenöl und/oder destilliertes Wasser.

20. Pflegender Vaginaltampon, umfassend die Vaginalpflegezusammensetzung gemäß einem der Ansprüche 1-10.

21. Pflegender Vaginaltampon gemäß Anspruch 20, umfassend 2 - 4, vorzugsweise 3 g der Vaginalpflegezusammensetzung pro Tampon, umfassend pro Gramm der Zusammensetzung:
# 10⁷ - 10⁹, vorzugsweise 10⁸ Keime Lactobacillus bifidus/g, 5%,
# 1 - 10, vorzugsweise 2,5 Gew % Saccharomaces cerevisiae,
# 0,05 - 0,3, vorzugsweise 0,1 Gew % Retinol-Acetat,
# 1 bis 3, vorzugsweise 1,5 Gew % D-alpha-Tocopherol,
# 1 - 6, vorzugsweise 0,8 Gew % Zink,
# 1 - 5, vorzugsweise 2 Gew % Melaleuca aetheroleum, und/oder
# 2 - 20, vorzugsweise 3 Gew % Oligofructose.

22. Pflegender Vaginaltampon gemäß einem der Ansprüche 20 und/oder 21, umfassend eine flüssigkeitsableitende äußere Schicht und eine innere, vorzugsweise quellfähige Speicherschicht.

23. Pflegender Vaginaltampon gemäß einem oder mehreren der Ansprüche 20 bis 22, wobei die Vaginalpflegezusammensetzung auf eine oberflächliche Schicht des Tampons durch Sprüh- oder Tauchverfahren aufgebracht wird oder optional den gesamten Tampon durchdringt.

24. Pflegender Vaginaltampon gemäß einem oder mehreren der Ansprüche 20 bis 23, wobei die Vaginalpflegezusammensetzung ausschließlich in einer Schicht direkt unter der äußeren Deckschicht des Tampons enthalten ist.

25. Pflegender Vaginaltampon gemäß einem oder mehreren der Ansprüche 20 bis 24, wobei die Vaginalpflegezusammensetzung durch Feuchtigkeit aktiviert wird.

26. Pflegende Slipeinlage, umfassend die Vaginalpflegezusammensetzung gemäß einem der Ansprüche 1 bis 10.

27. Pflegende Slipeinlage gemäß Anspruch 26, umfassend 2 - 5 g, vorzugsweise 3g der Vaginalpflegezusammensetzung pro Slipeinlage, wobei 1 g der Zusammensetzung umfasst:
# 10⁷ - 10⁹, vorzugsweise 10⁸ Keime Lactobacillus bifidus/g, 5%,
# 1 - 10, vorzugsweise 2,5 Gew % Saccharomaces cerevisiae,
# 0,05 - 0,3, vorzugsweise 0,1 Gew % Retinol-Acetat,
# 1 bis 3, vorzugsweise 1,5 Gew % D-alpha-Tocopherol,
# 1 - 6, vorzugsweise 3 Gew % Zink, und/oder
# 2 - 20, vorzugsweise 3 Gew % Oligofructose.

28. Pflegende Slipeinlage gemäß Anspruch 26 und/oder 27, weiterhin bestehend aus den üblichen Materialien für Slipeinlagen, insbesondere einer Deckschicht, einer die Flüssigkeit ableitenden Schicht, einer Speicherschicht und einer Rückschicht.

29. Pflegende Slipeinlage gemäß einem oder mehreren der Ansprüche 26 bis 28, wobei die Vaginalpflegezusammensetzung durch ein Sprühverfahren auf eine obere Schicht aufgebracht wird oder ein oder mehrere weitere Schichten damit versehen sind.

30. Pflegende Slipeinlage gemäß einem oder mehreren der Ansprüche 26 bis 29, wobei die Vaginalpflegezusammensetzung ausschließlich in einer Schicht direkt unter der Deckschicht der Slipeinlage enthalten ist.

31. Pflegende Slipeinlage gemäß einem oder mehreren der Ansprüche 26 - 30, wobei die Vaginalpflegezusammensetzung durch Feuchtigkeit aktiviert wird.

## Claims

1. Vaginal care composition, comprising viable Lactobacillus and/or Bifido bacterium microorganisms, preferably Lactobacillus bifidus; non-viable Saccharomyces cultures, preferably Saccharomyces cerevisiae; saccharide(s); vitamin A/retinol, and zinc.

2. Vaginal care composition according to claim 1, **characterised in that** it additionally comprises selenium, especially selenium-containing yeast, preferably in an amount of from 0.001 to 0.06 wt.% selenium.

3. Vaginal care composition according to claim 1 and/or 2, **characterised in that** it additionally comprises Melaleuca aetheroleum, preferably in an amount of from 0.5 to 10 wt.%, and/or camomile flower extract, preferably in an amount of from 0.01 to 5 wt.%.

4. Vaginal care composition according to one or more of claims 1 to 3, **characterised in that** it additionally comprises D-alpha-tocopherol, preferably in an amount of from 0.5 to 5 wt.%, and/or pantothenate, preferably in an amount of from 0.1 to 3 wt.%.

5. Vaginal care composition according to one or more of the preceding claims, **characterised in that** it additionally comprises fragrances, preferably natural fragrances, particularly preferably lavender oil in an amount of from 0.5 to 10 wt.%.

6. Vaginal care composition according to one or more of the preceding claims, **characterised in that** it comprises cocoa butter or a hard-fat carrier as base.

7. Vaginal care composition according to claim 1, **characterised in that** it comprises the constituents in the following amounts:
- Lactobacillus/Bifido bacterium: from 10⁷ to 10¹⁰ microorganisms/g of composition, 5 %
- Saccharomyces: from 0.1 to 40 wt.%
- saccharide(s): from 1 to 40 wt.%
- vitamin A: from 0.01 to 0.5 wt.%
- zinc: from 0.2 to 10 wt.%.

8. Vaginal care composition according to one or more of the preceding claims, wherein the saccharide (saccharides) is (are) selected from: oligofructose, inulin and/or lactose, preference being given to oligofructose.

9. Vaginal care composition according to one or more of the preceding claims, wherein the zinc is used in the form of zinc sulfate and/or zinc gluconate.

10. Vaginal care composition according to one or more of the preceding claims, wherein the vitamin A is used in the form of retinyl acetate and/or retinyl gluconate.

11. Use of the vaginal care composition according to one or more of the preceding claims for the care of the vaginal mucosa and for preventing infections thereof by bacteria, fungi and/or viruses.

12. Use of the vaginal care composition according to claim 11, wherein the composition is applied directly to the vaginal mucosa.

13. Caring ovule, comprising the vaginal care composition according to any one of claims 1 to 10.

14. Caring ovule according to claim 13, having a weight of 3 g per ovule, comprising per gram:
# from 10⁷ to 10⁹, preferably 10⁸, Lactobacillus bifidus microorganisms/g, 5 %,
# from 1 to 10 wt.%, preferably 2.5 wt.%, Saccharomyces cerevisiae,
# from 0.05 to 0.3 wt.%, preferably 0.1 wt.%, vitamin A,
# from 1 to 3 wt.%, preferably 1.8 wt.%, D-alpha-tocopherol,
# from 0.4 to 1.5 wt.%, preferably 0.9 wt.%, pantothenate,
# from 0.5 to 5 wt.%, preferably 0.8 wt.%, zinc,
# from 0.002 to 0.02 wt.%, preferably 0.005 wt.%, selenium,
# from 1 to 5 wt.%, preferably 3 wt.%, Melaleuca aetheroleum,
# from 1 to 5 wt.%, preferably 3 wt.%, lavender oil,
# from 0.2 to 3 wt.%, preferably 1 wt.%, camomile flower oil extract and/or
# from 2 to 20 wt.%, preferably 5 wt.%, oligofructose.

15. Caring ovule according to claim 13 and/or 14, wherein the ovule is composed on the basis of coconut oil, glycerol-gelatin or polyethylene glycol.

16. Caring ovule according to one or more of claims 13 to 15, wherein the ovule further comprises conventional ovule constituents, especially cocoa butter or hard fats.

17. Care cream, comprising the vaginal composition according to any one of claims 1 to 10.

18. Care cream according to claim 17, comprising
# from 10⁷ to 10⁹, preferably 10⁸, Lactobacillus bifidus microorganisms/g, 5 %,
# from 1 to 10 wt.%, preferably 2.5 wt.%, Saccharomyces cerevisiae,
# from 0.05 to 0.3 wt.%, preferably 0.1 wt.%, vitamin A,
# from 1 to 3 wt.%, preferably 1.5 wt.%, D-alpha-tocopherol,
# from 0.4 to 1.5 wt.%, preferably 0.7 wt.%, pantothenate,
# from 1 to 6 wt.%, preferably 3 wt.%, zinc,
# from 1 to 5 wt.%, preferably 2 wt.%, Melaleuca aetheroleum,
# from 0.05 to 3 wt.%, preferably 1 wt.%, camomile flower extract,
# from 2 to 20 wt.%, preferably 5 wt.%, oligofructose
# and/or from 1 to 5 wt.%, preferably 3 wt.%, lavender oil.

19. Care cream according to claim 17 and/or 18, wherein the cream further comprises conventional cream or ointment constituents, especially constituents selected from the following group: sunflower oil, Tegomuls 90 S, cetyl alcohol, Cetaceum artificiale, Polysorbat 80, methyl 4-hydroxybenzoate, propyl 4-hydroxybenzoate, sesame oil, wool wax, yellow wax, wool wax alcohols, glycerol, glycerol esters, palmitic acid ester, white Vaseline, liquid wax, olive oil and/or distilled water.

20. Caring vaginal tampon, comprising the vaginal care composition according to any one of claims 1 to 10.

21. Caring vaginal tampon according to claim 20, comprising from 2 to 4 g, preferably 3 g, of the vaginal care composition per tampon, comprising per gram of the composition:
# from 10⁷ to 10⁹, preferably 10⁸, Lactobacillus bifidus microorganisms/g, 5 %,
# from 1 to 10 wt.%, preferably 2.5 wt.%, Saccharomyces cerevisiae,
# from 0.05 to 0.3 wt.%, preferably 0.1 wt.%, retinol acetate,
# from 1 to 3 wt.%, preferably 1.5 wt.%, D-alpha-tocopherol,
# from 1 to 6 wt.%, preferably 0.8 wt.%, zinc,
# from 1 to 5 wt.%, preferably 2 wt.%, Melaleuca aetheroleum and/or
# from 2 to 20 wt.%, preferably 3 wt.%, oligofructose.

22. Caring vaginal tampon according to any one of claims 20 and/or 21, comprising a liquid-repellent outer layer and an inner, preferably swellable, storage layer.

23. Caring vaginal tampon according to one or more of claims 20 to 22, wherein the vaginal care composition is applied to a superficial layer of the tampon by spraying or immersing processes or optionally penetrates the entire tampon.

24. Caring vaginal tampon according to one or more of claims 20 to 23, wherein the vaginal care composition is contained solely in a layer directly beneath the outer cover layer of the tampon.

25. Caring vaginal tampon according to one or more of claims 20 to 24, wherein the vaginal care composition is activated by moisture.

26. Caring panty liner, comprising the vaginal care composition according to any one of claims 1 to 10.

27. Caring panty liner according to claim 26, comprising from 2 to 5 g, preferably 3 g, of the vaginal care composition per panty liner, 1 g of the composition comprising:
# from 10⁷ to 10⁹, preferably 10⁸, Lactobacillus bifidus microorganisms/g, 5 %,
# from 1 to 10 wt.%, preferably 2.5 wt.%, Saccharomyces cerevisiae,
# from 0.05 to 0.3 wt.%, preferably 0.1 wt.%, retinol acetate,
# from 1 to 3 wt.%, preferably 1.5 wt.%, D-alpha-tocopherol,
# from 1 to 6 wt.%, preferably 3 wt.%, zinc and/or
# from 2 to 20 wt.%, preferably 3 wt.%, oligofructose.

28. Caring panty liner according to claim 26 and/or 27, further comprising the conventional materials for panty liners, especially a cover layer, a liquid-repelling layer, a storage layer and a backing layer.

29. Caring panty liner according to one or more of claims 26 to 28, wherein the vaginal care composition is applied to an upper layer by a spraying process or one or more further layers are provided therewith.

30. Caring panty liner according to one or more of claims 26 to 29, wherein the vaginal care composition is contained solely in a layer directly beneath the cover layer of the panty liner.

31. Caring panty liner according to one or more of claims 26 to 30, wherein the vaginal care composition is activated by moisture.

## Revendications

1. Composition pour un traitement vaginal, comprenant des Lactobacillus viables et/ou des germes viables de bifidobactéries, de préférence Lactobacillus bifidus ; des cultures de Saccharomyces non-viables, de préférence Saccharomyces cerevisiae ; un ou des saccharide(s) ; de la vitamine A (rétinol) et du zinc.

2. Composition pour un traitement vaginal selon la revendication 1, **caractérisée en ce qu'**elle contient en outre du sélénium, en particulier une levure contenant du sélénium, de préférence une quantité de 0,001 à 0,06 pour cent en poids de sélénium.

3. Composition pour un traitement vaginal selon la revendication 1 et/ou la revendication 2, **caractérisée en ce qu'**elle contient de plus du Melaleuca aetheroleum, de préférence en une quantité de 0,5 à 10 pour cent en poids et/ou un extrait de fleurs de camomille, de préférence en une quantité de 0,01 à 5 pour cent en poids.

4. Composition pour un traitement vaginal selon au moins l'une des revendications 1 à 3, **caractérisée en ce qu'**elle contient en outre du D-alpha-tocophérol, de préférence en une quantité de 0,5 à 5 pour cent en poids, et/ou du pantothénate, de préférence en une quantité de 0,1 à 3 pour cent en poids.

5. Composition pour un traitement vaginal selon au moins l'une des revendications précédentes, **caractérisée en ce qu'**elle contient de plus des substances olfactives, de préférence des parfums naturels, en particulier de l'huile de lavande, en une quantité de 0,5 à 10 pour cent en poids.

6. Composition pour un traitement vaginal selon au moins l'une des revendications précédentes, **caractérisée en ce qu'**elle comprend en tant que base du beurre de cacao ou un support de matière grasse dure.

7. Composition pour un traitement vaginal selon la revendication 1, **caractérisée en ce qu'**elle comprend les composants dans les quantités suivantes :
- Lactobacillus/bifidobactéries : 10⁷ à 10¹⁰ germes/g de la composition, 5 %
- Saccharomyces : 0,1 à 40 pour cent en poids
- Saccharide(s) : 1 à 40 pour cent en poids
- Vitamine A : 0,01 à 0,5 pour cent en poids
- Zinc : 0,2 à 10 pour cent en poids.

8. Composition pour un traitement vaginal selon au moins l'une des revendications précédentes, dans laquelle le saccharide (les saccharides) est choisi parmi : l'oligofructose, l'insuline et/ou le lactose, avec une préférence pour l'oligofructose.

9. Composition pour un traitement vaginal selon au moins l'une des revendications précédentes, dans laquelle le zinc est utilisé sous forme de sulfate de zinc et/ou de gluconate de zinc.

10. Composition pour un traitement vaginal selon au moins l'une des revendications précédentes, dans laquelle la vitamine A est utilisée sous forme d'acétate de rétinyle et/ou de gluconate de rétinyle.

11. Utilisation de la composition pour un traitement vaginal selon au moins l'une des revendications précédentes pour le traitement de la muqueuse vaginale et la prévention des infections de celle-ci par des bactéries, des champignons et/ou des virus.

12. Utilisation de la composition pour un traitement vaginal selon la revendication 11, dans laquelle la composition est appliquée directement sur la muqueuse vaginale.

13. Ovule de soin, comprenant la composition pour un traitement vaginal selon l'une des revendications 1 à 10.

14. Ovule de soin, selon la revendication 13, présentant un poids de 3 g par ovule, comprenant par gramme :
# de 10⁷ à 10⁹, de préférence 10⁸, germes de Lactobacillus bifidus/g, 5 %,
# de 1 à 10, de préférence 2,5, % en poids de Saccharomyces cerevisiae,
# de 0,05 à 0,3, de préférence 0,1, % en poids de vitamine A,
# de 1 à 3, de préférence 1,8, % en poids de D-alpha-tocophérol ,
# de 0,4 à 1,5, de préférence 0,9, % en poids de pantothénate,
# de 0,5 à 5, de préférence 0,8, % en poids de zinc,
# de 0,002 à 0,02, de préférence 0,005, % en poids de sélénium,
# de 1 à 5, de préférence 3, % en poids de Melaleuca aetheroleum,
# de 1 à 5, de préférence 3, % en poids d'huile de lavande,
# de 0,2 à 3, de préférence 1, % en poids d'extrait de fleurs de camomille et/ou
# de 2 à 20, de préférence 5, % en poids d'oligofructose.

15. Ovule de soin selon la revendication 13 et/ou la revendication 14, lequel ovule est réalisé à base de graisse de coco, de glycérine-gélatine ou de polyéthylène glycol.

16. Ovule de soin selon au moins l'une des revendications 13 à 15, lequel ovule comprend en outre des composants conventionnels d'ovules, en particulier du beurre de cacao ou des graisses dures.

17. Crème de soin, comprenant la composition pour un traitement vaginal selon l'une des revendications 1 à 10.

18. Crème de soin selon la revendication 17 comprenant
# de 10⁷ à 10⁹, de préférence 10⁸, germes de Lactobacillus bifidus/g, 5 %,
# de 1 à 10, de préférence 2,5, % en poids de Saccharomyces cerevisiae,
# de 0,05 à 0,3 % en poids, de préférence 0,1 % en poids, de vitamine A,
# de 1 à 3 % en poids, de préférence 1,5 % en poids, de D-alpha-tocophérol,
# de 0,4 à 1,5 % en poids, de préférence 0,7 % en poids, de pantothénate,
# de 1 à 6 % en poids, de préférence 3 % en poids, de zinc,
# de 1 à 5 % en poids, de préférence 2 % en poids, de Melaleuca aetheroleum,
# de 0,05 à 3 % en poids , de préférence 1 % en poids, d'extrait de fleurs de camomille,
# de 2 à 20, de préférence 5, % en poids d'oligofructose,
# et/ou de 1 à 5 % en poids, de préférence 3 % en poids, d'huile de lavande.

19. Crème de soin selon la revendication 17 et/ou la revendication 18, dans laquelle la crème comprend en outre des composants habituels de crèmes ou de pommades, en particulier des composants choisis dans le groupe suivant : huile de tournesol, Tegomuls 90 S, alcool cétylique, Cetaceum artificiale, Polysorbat 80, hydroxy-4benzoate de méthyle, hydroxy-4benzoate de propyle, huile de sésame, lanoline, cire jaune, alcools de lanoline, glycérine, ester de glycérine, ester d'acide palmitique, vaseline blanche, cire très fluide, huile d'olive et/ou eau distillée.

20. Tampon vaginal de soin, comprenant la composition pour un traitement vaginal selon l'une des revendications 1 à 10.

21. Tampon vaginal de soin selon la revendication 20, comprenant de 2 à 4, de préférence 3, g de la composition pour un traitement vaginal par tampon, comprenant par gramme de la composition :
# de 10⁷ à 10⁹, de préférence 10⁸, germes de Lactobacillus bifidus/g, 5 %,
# de 1 à 10, de préférence 2,5, % en poids de Saccharomyces cerevisiae,
# de 0,05 à 0,3, de préférence 0,1, % en poids d'acétate de rétinol,
# de 1 à 3, de préférence 1,5, % en poids de D-alpha-tocophérol,
# de 1 à 6, de préférence 0,8, % en poids de zinc,
# de 1 à 5, de préférence 2, % en poids de Melaleuca aetheroleum, et/ou
# de 2 à 20, de préférence 3, % en poids d'oligofructose.

22. Tampon vaginal de soin selon la revendication 20 et/ou la revendication 21, comprenant une couche externe d'évacuation de fluide et une couche interne de rétention de préférence apte au gonflement.

23. Tampon vaginal de soin selon au moins l'une des revendications 20 à 22, dans lequel la composition pour un traitement vaginal est appliquée par un procédé de pulvérisation ou d'immersion sur une couche superficielle du tampon ou pénètre éventuellement dans l'ensemble du tampon.

24. Tampon vaginal de soin selon au moins l'une des revendications 20 à 23, dans lequel la composition pour un traitement vaginal est comprise exclusivement dans une couche directement située en dessous de la couche protectrice externe du tampon.

25. Tampon vaginal de soin selon au moins l'une des revendications 20 à 24, dans lequel la composition pour un traitement vaginal est activée par l'humidité.

26. Protège-slip de soin comprenant la composition pour un traitement vaginal selon l'une des revendications 1 à 10.

27. Protège-slip de soin selon la revendication 26, comprenant de 2 à 5 g, de préférence 3 g, de la composition pour un traitement vaginal par protège-slip, dans lequel 1 g de la composition comprend :
# de 10⁷ à 10⁹, de préférence 10⁸, germes de Lactobacillus bifidus/g, 5 %,
# de 1 à 10, de préférence 2,5, % en poids de Saccharomyces cerevisiae,
# de 0,05 à 0,3, de préférence 0,1, % en poids d'acétate de rétinol,
# de 1 à 3, de préférence 1,5, % en poids de D-alpha-tocophérol,
# de 1 à 6, de préférence 3, % en poids de zinc, et/ou
# de 2 à 20, de préférence 3, % en poids d'oligofructose.

28. Protège-slip de soin selon la revendication 26 et/ou la revendication 27, comprenant en outre des matières habituelles pour protège-slips, en particulier une couche protectrice, une couche d'évacuation de fluide, une couche de rétention et une couche de revers.

29. Protège-slip de soin selon au moins l'une des revendications 26 à 28, dans lequel la composition pour un traitement vaginal est appliquée par un procédé de pulvérisation sur une couche supérieure, ou une ou plusieurs autres couches en sont pourvues.

30. Protège-slip de soin selon au moins l'une des revendications 26 à 29, dans lequel la composition pour un traitement vaginal est comprise exclusivement dans une couche directement située en dessous de la couche protectrice du protège-slip.

31. Protège-slip de soin selon au moins l'une des revendications 26 à 30, dans lequel la composition pour un traitement vaginal est activée par l'humidité.
